# EUROPEAN PATENT SPECIFICATION

(11) **EP 1 909 741 B1**
(45) Date of publication and mention of the grant of the patent: **10.11.2010**
(21) Application number: 06765126.5
(22) Date of filing: 27.07.2006
(51) Int. Cl.: A61K 6/00

(54) **denture support composition**
Haftmittelzusammensetzung für Zahnprothesen
COMPOSITION

(30) Priority: 29.07.2005 GB 0515603
(43) Date of publication of application: 16.04.2008
(73) Proprietor: Reckitt Benckiser (UK) Limited, Slough, Berkshire SL1 3UH (GB)
(72) Inventor: DICKSON, P.A., Reckitt Benckiser Healthcare Ltd., Hull HU8 7DS (GB); LUCAS, R.A., Reckitt Benckiser Healthcare Ltd., Hull HU8 7DS (GB); SPENCER, A.V., Reckitt Benckiser Healthccare Ltd., Hull HU8 7DS (GB)
(74) Representative: Cawdell, Karen Teresa
(86) International application number: PCT/GB2006/002805
(87) International publication number: WO 2007/012859

(56) References cited:
- WO-A-01/41711
- WO-A-91/03987
- WO-A-91/14733
- US-A- 3 833 518
- US-A- 3 868 340
- US-A- 4 433 958
- US-A- 5 011 868
- US-A- 5 830 933

## Description

The present invention relates to a composition and method for supporting dentures within a user's mouth.

Denture wearers usually use denture fixative compositions (fixatives) to secure their dentures in place. Fixatives are widely available, but users encounter various problems with such products. For example many are water-soluble, leading to dissolution of the fixative over time, and loosening of the dentures. On the other hand, if the dentures are fixed too securely to the mouth, they cannot easily be removed. This can cause problems if, for example, small particles of food become stuck between the denture and the gum causing discomfort: if the denture cannot easily be removed, the discomfort cannot be alleviated. In addition, denture fixative compositions which do not allow easy removal may cause pain as they are pulled away from the mucosal surface to which they have become strongly adhered.

US-A-5011868 describes a denture adhesive base composition comprising a substantially anhydrous mixture of a cationic acrylamide polymer, sodium carboxymethylcellulose and ethylene oxide polymer.

An alternative method of securing dentures within the mouth is to provide an adhesive patch which sticks to both the mouth and the denture surface. For example, US 4503116 describes a dental adhesive device comprising a laminate of webs which are bonded together by thermoplastic ethylene oxide polymer. However such a patch is not always effective and provides no comfort benefit to the denture wearer.

A further problem suffered by denture wearers is that dentures may be ill-fitting or, when new, may take time to become comfortable in the mouth. Such problems are especially experienced by new users of dentures. A method sometimes employed by dentists when a person is first supplied with dentures is to provide a permanent cushioning pad which seats them comfortably in the mouth. However, this is not removable for the first few months, which introduces difficulties and drawbacks.

It is an object of the present invention to overcome or reduce at least one or more of the aforementioned disadvantages of present methods of retaining dentures within a user's mouth.

It is a further object of the present invention to provide for comfortable retention of dentures within a user's mouth.

According to a first aspect of the present invention there is provided a denture support composition in the form of a gel, the composition comprising:
a cationic polymer present in the composition in an amount of at least 0.5 wt% and up to 4 wt%;
an anionic polymer present in the composition in an amount of at least 0.1 wt% and up to 5 wt%; and
a carrier;
   wherein the composition is substantially insoluble in water.

The denture support composition of the invention is preferably one which allows a denture (i.e. a denture plate) to be retained within the mouth in such a manner that they may easily be removed. Thus, once in position dentures can easily be removed from the mouth and repositioned if desired. This may be done after any period of time, i.e. there is no need to wait for the composition to dissolve or loosen.

Equally, dentures may be left in place for long periods, without becoming loose or dislodged. For example they may be left in place overnight. This may be of benefit in maintaining the shape of the oral cavity.

Suitably the composition has a higher affinity with the material from which the dentures are made than it does with the mucosal surface of the mouth. Thus when the dentures are removed, the composition is preferably carried on the dentures and in preferred embodiments substantially none of the composition remains within the mouth.

The gel can be spread over the denture surface and the dentures can then be placed in position in the mouth. Upon contact with saliva, the composition preferably absorbs water and may take on a putty-like and/or springy consistency.

Preferably the composition of the present invention is not a fixative composition, although dentures are held securely in place within the mouth. Unlike some of the compositions of the prior art in which dentures are fixed in one place and often cannot be moved for several hours, dentures secured within the mouth using the composition of the present invention may be removed or repositioned immediately. The composition of the invention is better termed a denture support (or cushioning) composition than a denture fixative.

The composition of the present invention is substantially insoluble in water. By substantially insoluble is meant that the composition has a solubility in water at 25°C of less than 100 gdm⁻³, preferably less than 10 gdm⁻³, more preferably less than 5 gdm⁻³, and most preferably less than 1 gdm⁻³.

Preferably the solubility of the composition is such that after being left in the mouth for a period of 6 hours, less than 10% of the composition dissolves, and more preferably less than 5%.

Preferably, upon contact with an aqueous environment, the composition may absorb water and remain as a swollen gel. Water may replace the carrier in the composition and the polymer may become less soluble in water. Thus it does not wash away but remains swollen and able to provide support and/or cushioning.

The cationic polymer has cationic functional groups and the anionic polymer has anionic functional groups; and such groups have an affinity for each other. Preferably this affinity leads to an interaction or association between the two polymers. This interaction may result from, for example, Van der Waals forces, dipole-dipole interactions, hydrogen bonding or ionic interactions. Preferably there are hydrogen bonds and/or an ionic interactions and/or dipole-dipole interactions between the two polymers.

Preferably the cationic functional groups and anionic functional groups are not present in an equal molar ratio. Preferably there is a molar excess of one functional group.

Most preferably the composition of the present invention comprises a copolymer salt, formed from an anionic polymer and a cationic polymer.

The cationic polymer may comprise, for example, a cationic polyacrylamide, a cationic carbohydrate, a cationic polyacrylate, a cationic polymethacrylate or a cationic polyurethane.

Preferably the cationic functional groups of the cationic polymer comprise quaternary nitrogen centres. Preferably the nitrogen is bonded directly to the polymer backbone, and also to the three alkyl groups. Preferably the alkyl groups comprise up to 4 carbon atoms, more preferably ethyl and especially methyl.

In preferred embodiments the quaternary nitrogen species are appended to the polymer backbone at a distance of every 2-8 carbon atoms.

Preferably the cationic polymer is based on a methacrylate polymer. Especially preferred is trimethylamino ethyl methacrylate polymer. This is available under the trade mark POLYQUAT 37.

Preferably the molecular weight of the first cationic polymer is in the range of 10,000 - 4.5 billion.

Preferably the cationic polymer is present in the composition in an amount of at least 0.5 wt%, preferably at least 1 wt%, more preferably at least 1.5 wt%.

Preferably the cationic polymer is present in the composition in an amount of up to 4 wt%, preferably up to 3 wt%, more preferably up to 2.5 wt%.

The anionic polymer preferably comprises carboxylate residues. In an especially preferred embodiment the anionic polymer comprises a polyacrylic polymer (which includes carbopol^{RTM} polymers). Most preferable is a polymer sold under the trademark Carbopol 971P.

The term anionic polymer is used herein to refer to the polymer as present in the final composition, in its deprotonated form, comprising carboxylate anions. Of course, the polymer may be supplied as an acid which is neutral, but it should be understood that this will form an anionic polymer.

Preferably when a polyacrylic acid used as the precursor of the anionic polymer in this invention it has a molecular weight of between 500,000 and 4.5 billion preferably between 2,000,000 and 4.5 billion.

Preferably a polyacrylic acid polymer is supplied as a free acid, and must be deprotonated at least partially to provide the anionic carboxylate residue.

Preferably therefore the composition of the present invention further comprises a base. Suitable, bases include tertiary amines. Preferred bases are those which are pharmaceutically acceptable. An especially preferred base is triethanolamine.

Preferably the molar concentration of the base is less than that of the anionic functional groups.

Preferably, when the anionic polymer comprises carboxylate functional groups, the molar ratio of the base to carboxylate is sufficient to deprotonate approximately 80% of carboxylate groups present.

Preferably the anionic polymer is present in the composition in an amount of at least 0.1 wt%, preferably at least 0.25 wt%, more preferably at least 0.4 wt%. Preferably the anionic polymer is present in the composition in an amount of less than 5 wt%, more preferably less than 1 wt%, most preferably less than 0.7 wt%.

Wt% herein denotes weight of the specified component/total composition weight, expressed as a percentage.

Preferably there is a molar excess of either the cationic functional groups of the cationic polymer or the anionic functional groups of the anionic polymer. Preferably there is an excess of the cationic functional groups of the cationic polymer.

Preferably the molar ratio of cationic functional groups to anionic functional groups is at least 1:1, preferably at least 1.2:1.

Preferably the molar ratio of cationic functional groups to anionic functional groups is less than 2:1, preferably less than 1.5:1, more preferably less than 1.4:1. An especially preferred ratio of cationic functional groups to anionic functional groups is 1.25:1.

Preferably the mass ratio of cationic polymer to anionic polymer is at least 1.5:1, preferably at least 2:1, more preferably at least 2.5:1, more preferably at least 3:1, and most preferably at least 3.5:1.

Preferably the mass ratio of cationic to anionic polymer is less than 10:1, more preferably less than 8:1, more preferably less than 5:1, and most preferably less than 4:1.

Suitably the polymers comprised in the composition of the present invention are suspended or solvated in a carrier. This may comprise any pharmaceutically acceptable carrier. Any suitable carrier can be used. Preferably the carrier is an organic compound miscible with water. Preferably the carrier comprises a polyhydric alcohol, for example a liquid grade of a polyalkylene glycol, especially polyethylene glycol. Most preferably it is a polyhydric monomeric alcohol, especially glycerol.

The carrier is preferably present in an amount of at least 70 wt%, preferably at least 80 wt%.

Water may also be present in the composition, in addition to another pharmaceutically acceptable carrier. Preferably the composition comprises less than 15 wt% water, more preferably less than 10 wt%. Preferably the composition comprises at least 2 wt% water, preferably at least 5 wt%.

The composition is preferably supplied in the form of a thick gel. The polymer is suitably solvated therein.

Optionally the composition of the present invention may comprise further excipients, for example a flavouring or colouring and the like.

Preferably the composition contains up to 4 wt% (total) of extracts from plants, able to provide a sensory and/or therapeutic benefit, for example by promoting healing or providing soothing. Suitable extracts may include mentha piperita, menthol, lemongrass, bitter fennel oil, sage oil, chamomile and clove oil. Synthetic components could be used instead of natural extracts, but natural extracts are preferred.

Preferably, the composition consists essentially of a cationic polymer, an anionic polymer, a base and a pharmaceutically acceptable carrier: any further components are preferably present only as minor cosmetic excipients (for example flavouring and colouring agents).

Preferably the composition of the present invention has a pH in the range of 5 to 9, more preferably the pH of the composition is between 6 and 8.

Clearly the compounds, grades and amounts selected should be such as to allow a gel to be formed.

According to a second aspect of the present invention there is provided a method of supporting dentures within the mouth, the method comprising the steps of:
a) applying to the desired area of the dentures a composition of the first aspect;
b) positioning the dentures as required within the mouth so that the composition provides the desired support or cushioning effect.

Suitably the composition may be such that it undergoes an increase in viscosity during the method of the invention, upon contact with an aqueous environment. In the method of the present invention this aqueous environment is provided within the mouth, by the saliva.

According to a third aspect of the present invention there is provided use of a composition of the first aspect in order to support dentures within the mouth.

According to a fourth aspect of the present invention there is provided a process for preparing a composition of the first aspect, the process comprising the steps of:
a) dissolution of the cationic polymer in a pharmaceutically acceptable carrier;
b) addition of the anionic polymer thereto and stirring for a first period of time;
c) addition of a base to the reaction mixture; and
d) stirring the mixture for a second period of time.

Optionally the process may comprise adding further excipients and stirring if necessary.

Preferably the first period of stirring in step b) is at least 10 minutes, more preferably at least 15 minutes. Preferably the period of stirring is less than 40 minutes, more preferably less than 30 minutes.

Preferably the second period of time specified in step d) is at least 10 minutes, more preferably at least 15 minutes. Preferably the period of stirring is less than 40 minutes, more preferably less than 30 minutes.

Preferably all steps of the process of the fifth aspect are carried out at ambient temperature.

The invention will now be further described by way of the following non-limiting examples.

### Example 1

A denture support composition was prepared comprising the following components:
Trimethyl amino ethyl methacrylate polymer (Polyquat 37) 1.93 wt%
Carbopol 971P 0.53 wt%
Triethanolamine 0.88 wt%
Glycerol 96.595 wt%
Mentha piperita 0.05 wt%
Menthol 0.01 wt%
Lemongrass mint flavour 0.005 wt%.

### Method:

• The Polyquat 37 was dissolved in the glycerol (stirring for 20 minutes at room temperature)
• The Carbopol was added and stirred for a further 20 minutes before adding the triethanolamine
• The mixture was stirred without heating for 30 minutes before adding the mentha piperita, menthol and lemongrass mint flavour
• The mixture was stirred for 30 minutes before being packaged.

The composition was found to provide excellent support and cushioning properties. The dentures were easily removed and carried on them the composition. The dentures could then be easily cleaned. The herbal components gave sensory and/or natural therapeutic benefits.

### Example 2

A denture support composition was prepared, in the manner described in Example 1, comprising the following components
Trimethyl amino ethyl methacrylate polymer (Polyquat 37) 1.93 wt%
Carbopol 971P 0.53 wt%
Triethanolamine 0.88 wt%
Glycerol 94.351 wt%
Mentha piperita 0.0985 wt%
Menthol 0.02 wt%
Chamomile extract 2.0 wt%
Sage oil 0.1 wt%
Bitter fennel oil 0.1 wt%

Again, good properties were obtained, and the herbal components gave sensory and/or natural therapeutic benefits.

## Claims

1. A denture support composition in the form of a gel, the composition comprising:
a cationic polymer present in the composition in an amount of at least 0.5 wt% and up to 4 wt%;
an anionic polymer present in the composition in an amount of at least 0.1 wt% and up to 5 wt%; and
a carrier;
wherein the composition is substantially insoluble in water.

2. A denture support composition as claimed in claim 1 wherein the composition absorbs water when it is located in the oral cavity.

3. A denture support composition as claimed in claim 1 or claim 2 in which the carrier is present in an amount of at least 70 wt%.

4. A denture support composition as claimed in any preceding claim in which the cationic polymer has cationic functional groups which comprise quaternary nitrogen centres.

5. A denture support composition as claimed in claim 4 in which the cationic polymer comprises a polymer of trimethylamino ethyl methacrylate chloride.

6. A denture support composition as claimed in any of claims 3 to 5 in which the anionic functional groups comprise carboxylate ions.

7. A denture support composition as claimed in claim 6 wherein the anionic polymer comprises a polyacrylic acid (as homopolymer or a copolymer) which has been at least partially deprotonated.

8. A denture support composition as claimed in any of claims 3 to 7, comprising 1-3 wt% of the cationic polymer and 0.25-1 wt% of the anionic polymer.

9. A denture support composition as claimed in any preceding claim in which there is a molar excess of cationic functional groups of the cationic polymer, relative to anionic functional groups of the anionic polymer.

10. A denture support composition as claimed in claim 9 in which an amine base is present.

11. A denture support composition as claimed in any preceding claim wherein the carrier is a polyhydric alcohol.

12. A method of supporting dentures within the mouth, the method comprising the steps of:
a) applying to the desired area of the denture a composition as claimed in any preceding claim;
b) positioning the dentures as required within the mouth so that the composition provides a supporting or cushioning effect.

13. A method as claimed in claim 12 wherein the composition permits the dentures to be removed and repositioned if desired.

14. Use of a composition of as claimed in any of claims 1 to 11 in order to support dentures within the mouth.

15. A process for preparing a composition as claimed in any of claims 1 to 11, the process comprising the steps of:
a) dissolution of the cationic polymer in a pharmaceutically acceptable carrier;
b) addition thereto of an acid precursor of the anionic polymer and stirring for a first period of time;
c) addition of a base to the reaction mixture to effect partial or total deprotonation of the acid precursor; and
d) stirring the mixture for a second period of time.

## Patentansprüche

1. Zusammensetzung in Form eines Gels zum Halten einer Zahnprothese, wobei die Zusammensetzung umfasst:
ein kationisches Polymer, das in der Zusammensetzung in einer Menge von mindestens 0,5 Gew.% und bis zu 4 Gew.% vorhanden ist;
ein anionisches Polymer, das in der Zusammensetzung in einer Menge von mindestens 0,1 Gew.% und bis zu 5 Gew.% vorhanden ist; und
einen Träger;
wobei die Zusammensetzung im Wesentlichen wasserunlöslich ist.

2. Zusammensetzung zum Halten einer Zahnprothese nach Anspruch 1, wobei die Zusammensetzung Wasser absorbiert, wenn sie in der Mundhöhle angeordnet ist.

3. Zusammensetzung zum Halten einer Zahnprothese nach Anspruch 1 oder 2, wobei der Träger in einer Menge von mindestens 70 Gew.% gegenwärtig ist.

4. Zusammensetzung zum Halten einer Zahnprothese nach einem der vorhergehenden Ansprüche, wobei das kationische Polymer kationische funktionelle Gruppen aufweist, die quartäre Stickstoffzentren umfassen.

5. Zusammensetzung zum Halten einer Zahnprothese nach Anspruch 4, wobei das kationische Polymer ein Polymer von Trimethylaminoethylmethacrylatchlorid umfasst.

6. Zusammensetzung zum Halten einer Zahnprothese nach einem der Ansprüche 3 bis 5, wobei die anionischen funktionellen Gruppen Carboxylat-Ionen umfassen.

7. Zusammensetzung zum Halten einer Zahnprothese nach Anspruch 6, wobei das anionische Polymer eine Polyacrlysäure (als Homopolymer oder ein Copolymer) umfasst, die wenigstens teilweise deprotoniert wurde.

8. Zusammensetzung zum Halten einer Zahnprothese nach einem der Ansprüche 3 bis 7, umfassend 1 bis 3 Gew.% des kationischen Polymers und 0,25 bis 1 Gew.% des anionischen Polymers.

9. Zusammensetzung zum Halten einer Zahnprothese nach einem der vorhergehenden Ansprüche, wobei ein molarer Überschuss von kationischen funktionellen Gruppen des kationischen Polymers in Bezug auf anionische funktionelle Gruppen des anionischen Polymers vorhanden ist.

10. Zusammensetzung zum Halten einer Zahnprothese nach Anspruch 9, wobei eine Aminbase gegenwärtig ist.

11. Zusammensetzung zum Halten einer Zahnprothese nach einem der vorhergehenden Ansprüche, wobei es sich bei dem Träger um mehrwertigen Alkohol handelt.

12. Verfahren zum Halten von Zahnprothesen im Mund, wobei das Verfahren die folgenden Schritte umfasst:
a) Auftragen einer Zusammensetzung nach einem der vorhergehenden Ansprüche auf den gewünschten Bereich der Zahnprothese;
b) Positionieren der Zahnprothesen nach Bedarf im Mund, so dass die Zusammensetzung eine Halte- oder Polsterungswirkung bereitstellt.

13. Verfahren nach Anspruch 12, wobei die Zusammensetzung es ermöglicht, die Zahnprothesen zu entfernen oder neu zu positionieren, falls gewünscht.

14. Verwendung einer Zusammensetzung nach einem der Ansprüche 1 bis 11, um Zahnprothesen im Mund zu halten.

15. Prozess zur Herstellung einer Zusammensetzung nach einem der Ansprüche 1 bis 11, wobei der Prozess die folgenden Schritte umfasst:
a) Lösen des kationischen Polymers in einem pharmazeutisch unbedenklichen Träger;
b) Hinzufügen eines Säurevorläufers des anionischen Polymers und Rühren für einen ersten Zeitraum;
c) Hinzufügen einer Base zum Reaktionsgemisch, um eine teilweise oder vollständige Deprotonierung des Säurevorläufers zu bewirken; und
d) Rühren des Gemisches für einen zweiten Zeitraum.

## Revendications

1. Composition de support de prothèse sous la forme d'un gel, la composition comprenant :
un polymère cationique présent dans la composition dans une quantité d'au moins 0,5 % en poids et pouvant atteindre 4 % en poids ;
un polymère anionique présent dans la composition dans une quantité d'au moins 0,1 % en poids et pouvant atteindre 5 % en poids ; et
un excipient;
dans laquelle la composition est sensiblement insoluble dans l'eau.

2. Composition de support de prothèse selon la revendication 1 dans laquelle la composition absorbe l'eau lorsqu'elle se trouve dans la cavité buccale.

3. Composition de support de prothèse selon la revendication 1 ou la revendication 2 dans laquelle l'excipient est présent dans une quantité d'au moins 70 % en poids.

4. Composition de support de prothèse selon l'une quelconque des revendications précédentes dans laquelle le polymère cationique comporte des groupes fonctionnels cationiques qui comprennent des centres d'azote quaternaire.

5. Composition de support de prothèse selon la revendication 4 dans laquelle le polymère cationique comprend un polymère de chlorure de triméthylaminoéthylméthacrylate.

6. Composition de support de prothèse selon l'une quelconque des revendications 3 à 5 dans laquelle les groupes fonctionnels anioniques comprennent des ions carboxylate.

7. Composition de support de prothèse selon la revendication 6 dans laquelle le polymère anionique comprend un acide polyacrylique (sous la forme d'un homopolymère ou d'un copolymère) qui a été au moins partiellement déprotoné.

8. Composition de support de prothèse selon l'une quelconque des revendications 3 à 7, comprenant de 1 à 3 % en poids du polymère cationique et de 0,25 à 1 % en poids du polymère anionique.

9. Composition de support de prothèse selon l'une quelconque des revendications précédentes dans laquelle il existe un excès molaire de groupes fonctionnels cationiques du polymère cationique, par rapport aux groupes fonctionnels anioniques du polymère anionique.

10. Composition de support de prothèse selon la revendication 9 dans laquelle une base aminée est présente.

11. Composition de support de prothèse selon l'une quelconque des revendications précédentes dans laquelle l'excipient est un alcool polyhydrique.

12. Procédé de support de prothèses dans la bouche, le procédé comprenant les étapes consistant à :
a) appliquer sur la zone souhaitée de la prothèse une composition selon l'une quelconque des revendications précédentes ;
b) placer les prothèses de manière adéquate dans la bouche de manière à ce que la composition ait un effet de support ou de tamponnage.

13. Procédé selon la revendication 12 dans lequel la composition permet de retirer et de replacer les prothèses si on le souhaite.

14. Utilisation d'une composition selon l'une quelconque des revendications 1 à 11 afin d'offrir un support aux prothèses dans la bouche.

15. Procédé de préparation d'une composition selon l'une quelconque des revendications 1 à 11, le procédé comprenant les étapes de :
a) dissolution du polymère cationique dans un excipient pharmaceutiquement acceptable ;
b) addition à ce mélange d'un précurseur acide du polymère anionique et agitation pendant une première période de temps ;
c) addition d'une base au mélange réactionnel pour effectuer une déprotonation partielle ou totale du précurseur acide ; et
d) agitation du mélange pendant une deuxième période de temps.
